# EUROPÄISCHE PATENTSCHRIFT

(11) **EP 1 619 239 B1**
(45) Veröffentlichungstag und Bekanntmachung des Hinweises auf die Patenterteilung: **16.04.2008**
(21) Anmeldenummer: 05015610.8
(22) Anmeldetag: 19.07.2005
(51) Int. Cl.: C12M 1/107

(54) **Biogasanlage zur Fermentation von organischen Stoffen**
Biogas plant for the fermentation of organic material
Installation de biogaz pour la fermentation de matèriaux organiques

(30) Priorität: 24.07.2004 DE 202004011561 U
(43) Veröffentlichungstag der Anmeldung: 25.01.2006
(73) Patentinhaber: UTS Biogastechnik GmbH, 84419 Obertaufkirchen (DE)
(72) Erfinder: Bürger, Adam, 84405 Grüntegernbach (DE)
(74) Vertreter: Liebl, Thomas

(56) Entgegenhaltungen:
- DE-A1- 4 120 988
- DE-C1- 4 015 478
- DE-U1- 20 110 792

## Beschreibung

Die Erfindung betrifft eine Biogasanlage zur Fermentation von organischen Stoffen nach dem Oberbegriff des Anspruchs 1.

In derartigen Biogasanlagen läuft ein Fermentationsprozess ab, bei dem organische Stoffe, wie z. B. Gras, Stalldung, Jauche, Stroh und dergleichen vergast werden. Dieser Fermentationsprozess findet in einem Fermenterbehälter statt, in dem die organischen Stoffe mit Flüssigkeit versetzt werden und der Fermentations- bzw. Vergasungsprozess unter aeroben oder anaeroben Bedingungen durch Mikroorganismen, wie z. B. Hefen, Bakterien, etc. durchgeführt wird. Die bei der Fermentation entstehenden Biogase sammeln sich in einem oberen Fermenterbehälterbereich und können direkt zur Energieerzeugung verwendet werden, z. B. als Heizgas zur Stromerzeugung in nachgeschalteten Brennkraftmaschinen mit Elektrogeneratoren. Allgemein bekannte Fermenterbehälter sind entweder im Erdboden eingelassen oder als Hochbehälter ausgeführt. Solche Biogasanlagen zur Fermentation von organischen Stoffen finden meist in der dezentralen Verarbeitung und Verwertung von organischen Reststoffen im landwirtschaftlichen Bereich Verwendung.

Für eine gleichmäßige Verteilung der Feststoffe in der Schlämmung und für das Verhindern des Festsetzens der Feststoffe an den Fermenterseitenwänden ist wenigstens ein Rührwerk als Umwälzeinrichtung im Fermenterbehälter erforderlich. Eine Biogasanlage zur Fermentation von organischen Stoffen mit einem Fermenterbehälter und einem Rührwerk, ist aus der DE 197 14 342 C2 bekannt. Im Fermenterbehälter ist hier ein elektrisches Tauchrührgerät mit einem relativ großen Elektromotor und drehangetriebenen Rührflügeln über eine Führungseinrichtung an einer vertikalen Stützstange höhenverstellbar gehalten. Für diese Höhenverstellung ist eine Seilwinde am oberen Stützstangenbereich sowie eine kraftübertragende Seilverbindung zum Tauchrührgerät vorgesehen. Ferner enthält die Fermenterdeckenwand eine Wartungsöffnung und einen darüber angebrachten Dom, durch die die Stützstange nach oben dicht herausgeführt ist. Durch eine Verschwenkung der Stützstange um die Stangenachse sind zudem unterschiedliche Winkelstellungen des Tauchrührgeräts möglich. Die vorstehende höhenverstellbare und winkelverstellbare Anordnung eines Tauchrührgeräts an einer zentralen Stützstange im Fermenterbehälter ist relativ aufwändig und teuer.

Weiter ist eine ähnliche gattungsgemäße Biogasanlage zur Fermentation von organischen Stoffen bekannt (DE 201 10 792.9 U1), bei der die Halte- und Stelleinrichtung für das wenigstens eine Tauchrührgerät als Schwingenanordnung ausgebildet ist. Dazu ist wenigstens ein Schwingen-Gelenklager mit etwa horizontaler Gelenklagerachse ortsfest im Fermenterbehälter insbesondere an einer Fermenterseitenwand angebracht. Mit diesem Schwingen-Gelenklager ist ein Schwingenarm insbesondere in der Art eines Schwingenrahmens schwenkbar verbunden. Das Tauchrührgerät ist im Bereich des freien Schwingenarmendes angebracht, so dass bei unterschiedlichen Schwenkpositionen des Schwingenarms entsprechend zugeordnete unterschiedliche Höhenpositionen des Tauchrührgeräts einstellbar sind. Die Rührflügel-Drehachse und die Gelenklagerachse liegen hier achsparallel.

Das Schwingen-Gelenklager ist so angeordnet und die Länge des Schwingenarms ist so gewählt, dass eine Verstellung des Tauchrührgeräts unmittelbar in den Bereich der Fermenterdeckenwand durchführbar ist. Dort ist auf diese Weise eine Wartungsposition für das Tauchrührgerät bei vollem oder leicht abgesenktem Fementerfüllstand geschaffen, wobei das Tauchrührgerät für Wartungszwecke durch eine dortige dicht verschließbare Wartungsöffnung in der Fermenterdeckenwand zugänglich ist. Diese Wartungsöffnung ist wenigstens so groß, dass das Tauchrührgerät durch die Öffnung hindurch montierbar und demontierbar ist. Diese Wartungsmöglichkeit kann nur bei Fermenterbehältern mit einer stabilen, begehbaren Fermenterdeckenwand eingerichtet werden und ist insbesondere bei Fermenterbehältern mit einem Foliendach nicht möglich. Zudem ist eine Wartung von oben durch eine geöffnete Wartungsöffnung nach unten an einem dort positionierten Tauchrührgerät beschwerlich und sicherheitstechnisch nur durch Zusatzmaßnahmen, wie eine Sicherung des Werkers gegen einen Fall kopfüber in den Behälter, zu beherrschen. Da eine Fermenterdeckenwand für ein Aufstellen oder Befahren mit Hubgeräten meist nicht ausgelegt ist, sind auch das Herausheben eines demontierten Tauchrührgeräts aus der Wartungsöffnung und der Abtransport beschwerlich und aufwendig.

Zudem ist eine Rühreinrichtung für einen Fermenter bekannt (DE 41 20 988 A1), bei der eine angetriebene Welle mit endseitigen Rührflügeln schwenkbar und gasdicht in aufwendiger Weise mittels eines Kugelkörpers und eines flexiblen Gummischlauchs durch eine Fermenterwand geführt ist.

Aufgabe der Erfindung ist es, eine gattungsgemäße Biogasanlage so weiterzubilden, dass eine Wartung, sowie eine Demontage und Wiedermontage einer Rühreinrichtung, insbesondere bei Fermenterbehältern mit einem Foliendach, mit weniger Aufwand möglich sind.

Diese Aufgabe wird mit den Merkmalen des Anspruchs 1 gelöst.

Gemäß Anspruch 1 ist im oberen Bereich der Fermenterseitenwand eine mittels eines Wartungsdeckels dicht und lösbar verschließbare, vertikal ausgerichtete Wartungsöffnung angebracht.

Das Arbeiten durch eine vertikal ausgerichtete Wartungsöffnung ist für einen Werker einfach und bequem und sicher möglich. Zudem ist bei Hochbehältern als auch bei teilweise im Erdboden eingelassenen Fermenterbehältern deren Seitenwandbereich und damit die Wartungsöffnung von außen für ein Hubgerät gut zugänglich, so dass eine Rühreinrichtung einfach aus dem Fermenterbehälter bei Bedarf herausgehoben und abtransportiert sowie wieder montiert werden.

Dazu ist das wenigstens eine Schwingen-Gelenklager lösbar im Seitenrandbereich der Wartungsöffnung, vorzugsweise an einem dort befestigten stabilen Halterahmen angebracht dergestalt, dass nach dem Lösen des wenigstens einen Schwingen-Gelenklagers der Schwingenarm mit dem daran angebrachten Drehantrieb und Rührflügel durch die Wartungsöffnung aus dem Fermenterbehälter heraus verlagerbar ist. Vorteilhaft sind hier die Schwingen-Gelenklager im Bereich der Wartungsöffnung für einen Werker besonders gut zugänglich, so dass diese bei einer Wartung einfach gelöst werden können und die ganze Einheit mit Schwingenarm einfach demontierbar ist. Vor dem Lösen des wenigstens einen Schwingen-Gelenklagers durch die Wartungsöffnung wird von außen in den Fermenterbehälter eine Stützvorrichtung, vorzugsweise an einem Traktorfrontend, eingebracht, mit der der Schwingenarm in einer etwa horizontalen Position gehalten wird. Eine Verlagerung aus dem Fermenterbehälter nach dem Lösen des wenigstens einen Schwingen-Gelenklagers und gegebenenfalls einer Halte- und Stelleinrichtung mittels dieser Stützvorrichtung ist besonders einfach und bequem möglich.

Ein besonderer Vorteil besteht weiter darin, dass die erfindungsgemäß Anordnung auch bei Fermenterbehältern mit nicht begehbaren Abdeckungen, insbesondere bei den derzeit bevorzugten Fermenterbehältern mit Foliendächern nach Anspruch 2 einfach, funktionsfähig und kostengünstig einsetzbar ist.

Für eine ausgeglichene Kraftabstützung insbesondere von Rührreaktionskräften wird mit Anspruch 3 vorgeschlagen, dass der Schwingenarm als rechteckiger oder dreieckiger Schwingenrahmen ausgebildet ist, wobei ein Rahmenteil an zwei beabstandet koaxial angeordneten Schwingen-Gelenklagern schwenkbar gelagert ist.

In Verbindung mit der Anordnung der Schwingen-Gelenklager im Bereich der Wartungsöffnung wird mit Anspruch 4 eine bevorzugte funktionsfähige und einfach montierbare Betätigungsvorrichtung mit einer hydraulischen oder pneumatischen Stellzylinder-Kolben-Einheit vorgeschlagen.

Der Drehantrieb soll nach Anspruch 5 für eine konstruktiv günstige Lösung als elektrischer, pneumatischer oder vorzugsweise hydraulischer Antriebsmotor am Schwingenarmende bzw. am Ende des Schwingenarmschenkels zusammen mit dem oder den Rührflügeln angeordnet sein.

Anhand einer Zeichnung wird die Erfindung näher erläutert.

Es zeigen:
- Fig. 1: eine Innenansicht eines Fermenterteilbereichs mit einer Rühreinrichtung,
- Fig. 2: eine Ausschnittvergrößerung aus Fig. 1, und
- Fig. 3: eine schematische Schnittdarstellung durch einen Teilbereich des Fermenters nach Fig. 1 mit demontierter Rühreinrichtung.

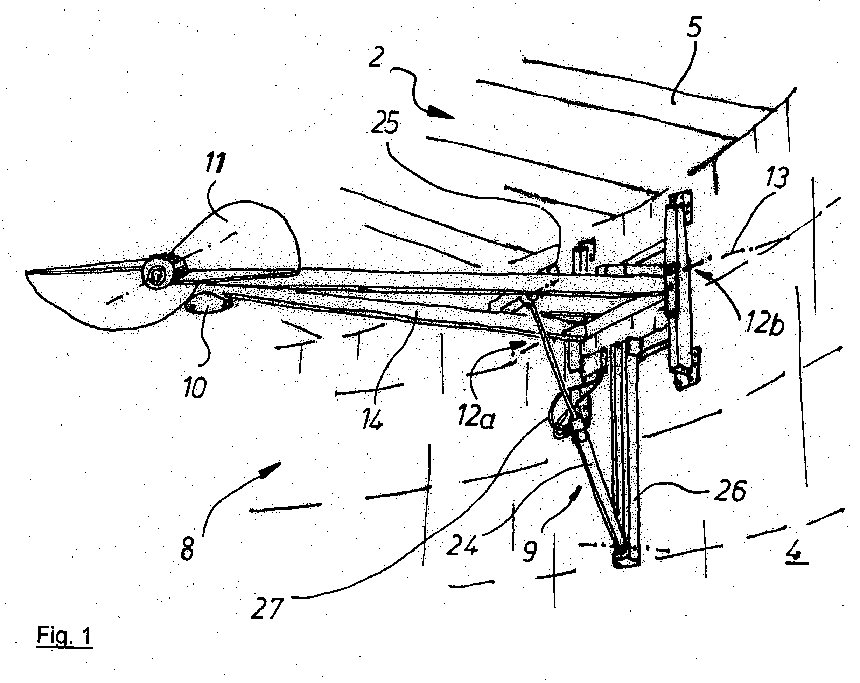

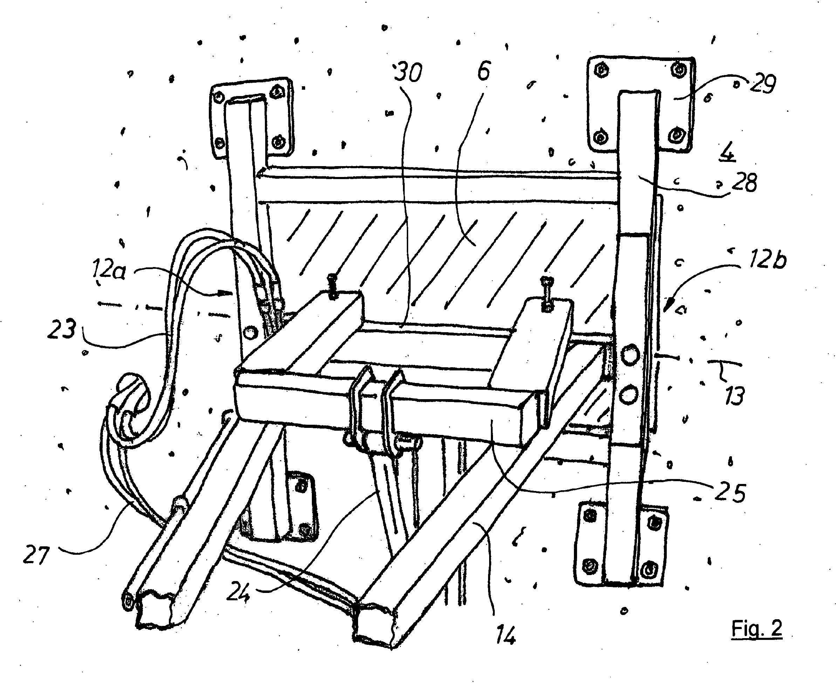

In den Fig. 1 und 2 ist jeweils in einer schematischen Schnittdarstellung ein Teilbereich eines Fermenters einer Biogasanlage zur Fermentation von organischen Stoffen dargestellt. Der Fermenter weist dabei einen Fermenterbehälter 2 mit einer Fermenterseitenwand 4 auf, der als teilweise in den Boden eingelassener Hochbehälter mit einem Foliendach 5 als Fermenterabdeckung ausgeführt ist.

In einem oberen Bereich der Fermenterseitenwand 4 ist eine Wartungsöffnung 6 angebracht, die mittels eines Wartungsdeckels dicht verschlossen werden kann. Im Fermenterbehälter 2 werden für den Fermentations- bzw. Vergasungsprozess organische Stoffe mit Flüssigkeit versetzt, wobei für einen optimierten Fermentations- bzw. Vergasungsprozess eine gleichmäßige Verteilung der organischen Feststoffe in der Schlämmung vorhanden sein soll. Oberhalb der Schlämmung sammeln sich die beim Fermentations- bzw. Vergasungsprozess entstehenden Biogase an, die von dort zur Energieerzeugung abgezogen werden können.

Um die gleichmäßige Verteilung der organischen Feststoffe in der Schlämmung sicherzustellen, weist der Fermenter eine Rühreinrichtung 8 und eine Halte- und Stelleinrichtung 9, die im Fermenterbehälter 2 befestigt ist, auf. Die Rühreinrichtung 8 besteht aus einem Drehantrieb 10 und einer zweiflügeligen Rührflügelanordnung mit zwei an einer Nabe gegenüberliegenden Rührflügeln 11. Die Halte- und Stelleinrichtung 9 ist als Schwingenanordnung ausgebildet, wobei ein Schwingen-Gelenklager 12 mit einer etwa horizontal verlaufenden Gelenklagerachse 13 im Fermenterbehälter 2 an der Fermenterseitenwand 4 angeordnet ist. An Schwingen-Gelenklagern 12a, 12b ist ein Schwingenarm 14 angebunden, der um die Gelenklagerachse 13 verschwenkt werden kann.

Der Schwingenarm 14 ist als dreieckiger Schwingenrahmen ausgebildet, wobei an einer Dreiecksspitze der Drehantriebe 10 mit den Rührflügeln 11 und an den Ecken eines Dreieckschenkels die Schwingengelenklager 12a, 12b angeordnet sind. Beim Drehantrieb 10 handelt es sich um einen Hydraulikmotor, der über Hydraulikleitungen 23 versorgt wird. Die Halte- und Stelleinrichtung besteht aus einer hydraulischen Stellzylinder-Kolben-Einheit 24, die einerseits oben an einer Querstrebe 25 des Schwingenarms 14 lösbar angelenkt ist und andererseits an der Fermenterseitenwand 4 mit einem unteren Ende in einer Vertikalschiene 26 nach oben ausschiebbar gelenkig abgestützt ist. Die hydraulische Stellzylinder-Kolben-Einheit 24 wird über Hydraulikleitungen 27 in unterschiedliche Ausfahrstellungen über ein externes Steuergerät betätigt, wodurch die Rührflügel 11 in unterschiedliche Höhen im Fermenterbehälter 2 verstellt werden. Die Rühreinrichtung 8 ist ersichtlich im oberen Bereich der Fermenterseitenwand 4 unterhalb einer Abdeckung beispielsweise unter einem Foliendach 5 angeordnet. In der Fermenterseitenwand 4 ist in diesem oberen Bereich eine rechteckige Wartungsöffnung 6 als Durchbruch angebracht, auf deren Innenseite ein Halterahmen 28 als Begrenzung des Durchbruchs stabil und dauerhaft mittels Halteblechen 29 angeschraubt ist.

Aus den Fig. 1 und 2 ist ersichtlich, dass der Schwingenarm 14 von außen her so in die Wartungsöffnung 6 eingeschoben ist, dass er mit seitlich überstehenden Enden eines Rahmenschenkels 30 und den dort angebrachten (hier verdeckten) Schwingen-Gelenklagern 12a, 12b von außen her an den vertikalen Rahmenteilen des Halterahmens 28 anliegt und lösbar befestigt ist.

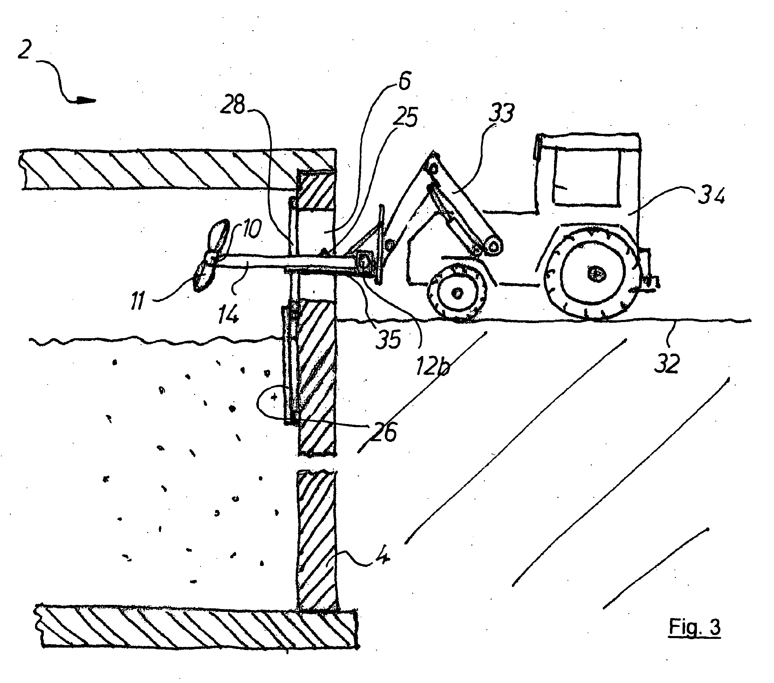

In Fig. 3 ist die einfache Montagemöglichkeit des Schwingenarms 14 mit dem Drehantrieb 10 und den Rührflügeln 11 dargestellt. Hier ist ein Vertikalschnitt durch einen Fermenterbehälter 2 dargestellt, der eine Abdeckung mittels einer Betondecke 31 aufweist und der mit seinen Fermenterseitenwänden 4 so in den Erdboden eingelassen ist, dass er mit einem Überstand das Bodenniveau 32 überragt. In diesem Überstand ist in der Fermenterseitenwand 4 die Wartungsöffnung 6 eingebracht, die in ihrer Höhe im Bereich einer Frontladevorrichtung 33 eines Traktors 34 liegt. Der Schwingenarm 14 mit seinen Schwingen-Gelenklagern 12a, 12b, mit dem Drehantrieb 10 und mit den Rührflügeln 11 ist in etwa horizontaler Ausrichtung an einer Ladegabel 35 der Frontladevorrichtung 33 befestigt und dieser Position in die Wartungsöffnung eingefahren bis die Schwingen-Gelenklager 18a, 18b am Halterahmen 28 anliegen. Nach der dortigen Befestigung der Schwingen-Gelenklager 12a, 12b wird die Stellzylinder-Kolben-Einheit 24 mit ihrem unteren Ende in die Vertikalschiene 26 eingeschoben und mit ihrem oberen Ende an der Querstrebe 25 des Schwingenarms 14 angeschlossen, wodurch der Schwingenarm 14 in seiner horizontalen Lage damit abgestützt ist. Danach ist die Abstützung durch die Frontladevorrichtung 33 entbehrlich und der Traktor 24 kann weggefahren werden. Anschließend wird ein (nicht dargestellter) Deckel zur Abdichtung von außen über der Wartungsöffnung 6 angebracht. Eine Demontage für Wartungs- und/oder Reparaturzwecke kann in umgekehrter Reihenfolge ebenso einfach und schnell durchgeführt werden.

## Patentansprüche

1. Biogasanlage zur Fermentation von organischen Stoffen,
mit einem Fermenter, der aus einem Fermenterbehälter mit einer Fermenterbodenwand, mit Fermenterseitenwänden und mit einer dichten Fermenterabdeckung sowie aus einer Rühreinrichtung besteht, wobei
die Rühreinrichtung wenigstens einen Rührflügel mit Drehantrieb und eine im Fermenterbehälter befestigte Halte- und Stelleinrichtung umfasst, die von außerhalb des Behälters über eine Betätigungseinrichtung in unterschiedlichen Höhenpositionen ein- und feststellbar ist, und
die Halte- und Stelleinrichtung als Schwingenanordnung ausgebildet ist, und dazu wenigstens ein Schwingen-Gelenklager mit etwa horizontaler Gelenklagerachse im Fermenterbehälter unmittelbar oder mittelbar an einer Fermenterseitenwand angebracht ist, mit dem Schwingen-Gelenklager ein Schwingenarm schwenkbar verbunden ist und der wenigstens eine Rührflügel im Bereich des freien Schwingenarmendes angebracht ist, so dass bei unterschiedlichen Schwenkpositionen des Schwingenarms entsprechend zugeordnete, unterschiedliche Höhenpositionen des Rührflügels einstellbar sind,
**dadurch gekennzeichnet,**
- **dass** im oberen Bereich der Fermenterseitenwand (4) eine mittels eines Wartungsdeckels dicht und lösbar verschließbare, vertikal ausgerichtete Wartungsöffnung (6) angebracht ist,
- **dass** das wenigstens eine Schwingen-Gelenklager (12a, 12b) lösbar im Seitenrandbereich der Wartungsöffnung (6), vorzugsweise an einem dort befestigten stabilen Halterahmen (28) angebracht ist dergestalt, dass nach dem Lösen des wenigstens einen Schwingen-Gelenklagers (12a, 12b) der Schwingenarm (14) mit dem daran angebrachten Drehantrieb (10) und Rührflügel (11) durch die Wartungsöffnung (6) aus dem Fermenterbehälter (2) heraus verlagerbar ist, wobei
- vor dem Lösen des wenigstens einen Schwingen-Gelenklagers (12a, 12b) durch die Wartungsöffnung (6) von außen in den Fermenterbehälter (2) eine Stützvorrichtung (33, 35), vorzugsweise an einem Traktorfrontend, einbringbar ist zur Halterung des Schwingenarms (14) in einer etwa horizontalen Position und zu dessen Verlagerung aus dem Fermenterbehäl-ter (2) nach dem Lösen des wenigstens einen Schwingen-Gelenklagers (12a, 12b).

2. Biogasanlage nach Anspruch 1, **dadurch gekennzeichnet, dass** der Fermenterbehälter (2) mit einem Foliendach (5) als Fermenterabdeckung ausgeführt ist.

3. Biogasanlage nach Anspruch 1 oder 2, **dadurch gekennzeichnet, dass** der Schwingenarm (14) als rechteckiger oder dreieckiger Schwingenrahmen ausgebildet ist, wobei ein Rahmenteil (30) an zwei beabstandet koaxial angeordneten Schwingen-Gelenklagern (12a, 12b) schwenkbar gelagert ist.

4. Biogasanlage nach einem der Ansprüche 1 bis 3, **dadurch gekennzeichnet, dass** die Betätigungseinrichtung eine hydraulische und/oder pneumatische Stellzylinder-Kolben-Einheit (24) aufweist, die einerseits am Schwingenarm (14), gegebenenfalls lösbar angelenkt ist und andererseits an der Fermenterseitenwand (4) unter dem Schwingenarm (14), gegebenenfalls aus einer Vertikalschiene (26) nach oben ausschiebbar, angelenkt ist.

5. Biogasanlage nach einem der Ansprüche 1 bis 4, **dadurch gekennzeichnet, dass** der Drehantrieb (10) als elektrischer, pneumatischer oder vorzugsweise hydraulischer Antriebsmotor am Schwingenarmende bzw. am Ende des Schwingenarmschenkels (15) zusammen mit dem oder den Rührflügeln (11) angeordnet ist.

## Claims

1. Biogas plant for the fermentation of organic materials,
with a fermenter which comprises a fermenter tank with a fermenter bottom wall, with fermenter side walls and with a tight fermenter covering, and comprises a stirring device, wherein
the stirring device comprises at least one stirring blade with a rotary actuator and a holding and adjusting device which is fastened in the fermenter tank and can be set and fixed in different height positions from outside the tank via an actuating device, and
the holding and adjusting device is designed as a rocker arrangement, and, for this purpose, at least one rocker-type pivot bearing with an approximately horizontal pivot-bearing axis is fitted in the fermenter tank directly or indirectly to a fermenter side wall, a rocker arm is connected pivotably to the rocker-type pivot bearing, and the at least one stirring blade is fitted in the region of the free rocker-arm end such that, at different pivoting positions of the rocker arm, correspondingly assigned, different height positions of the stirring blade can be set,
**characterized**
- **in that** a maintenance opening (6) which can be tightly and releasably closed by means of a maintenance cover and is oriented vertically is provided in the upper region of the fermenter side wall (4),
- **in that** the at least one rocker-type pivot bearing (12a, 12b) is fitted releasably in the side edge region of the maintenance opening (6), preferably to a stable holding frame (28) fastened there, in such a manner that, after the at least one rocker-type pivot bearing (12a, 12b) is released, the rocker arm (14) together with the rotary actuator (10) fitted thereon and stirring blade (11) can be displaced out of the fermenter tank (2) through the maintenance opening (6), with it being possible
- before the at least one rocker-type pivot bearing (12a, 12b) is released, for a supporting device (33, 35) to be introduced through the maintenance opening (6) from the outside into the fermenter tank (2), preferably on a front end of a tractor, in order to hold the rocker arm (14) in an approximately horizontal position and for the displacement thereof out of the fermenter tank (2) after the at least one rocker-type pivot bearing (12a, 12b) is released.

2. Biogas plant according to Claim 1, **characterized in that** the fermenter tank (2) is designed with a sheeting roof (5) as the fermenter covering.

3. Biogas plant according to Claim 1 or 2, **characterized in that** the rocker arm (14) is designed as a rectangular or triangular rocker frame, with one frame part (30) being mounted pivotably on two spaced-apart, coaxially arranged rocker-type pivot bearings (12a, 12b).

4. Biogas plant according to one of Claims 1 to 3, **characterized in that** the actuating device has a hydraulic and/or pneumatic adjusting cylinder-piston unit (24) which, firstly, is coupled, if appropriate releasably, to the rocker arm (14) and, secondly, is coupled to the fermenter side wall (4) under the rocker arm (14), if appropriate in a manner such that it can be pushed upwards out of a vertical rail (26).

5. Biogas plant according to one of Claims 1 to 4, **characterized in that** the rotary actuator (10) is arranged as an electric, pneumatic or preferably hydraulic driving motor at the rocker-arm end or at the end of the rocker-arm limb (15) together with the stirring blade or the stirring blades (11).

## Revendications

1. Installation de biogaz pour la fermentation de substances organiques,
avec un fermenteur qui se compose d'un réservoir de fermenteur doté d'une paroi de fond de fermenteur, de parois latérales de fermenteur et d'un élément de recouvrement de fermenteur étanche, ainsi que d'un dispositif de malaxage,
le dispositif de malaxage comprenant au moins une pale agitatrice avec un entraînement rotatif et un dispositif de commande et de maintien fixé dans le réservoir du fermenteur, lequel peut être réglé et fixé dans différentes positions verticales de l'extérieur du réservoir par le biais d'un dispositif d'actionnement, et
le dispositif de maintien et de commande étant réalisé comme un ensemble oscillant et à cet effet, au moins une articulation à rotule oscillante présentant un axe d'articulation à rotule à peu près horizontal dans le réservoir de fermenteur étant montée directement ou indirectement sur une paroi latérale de fermenteur, à l'articulation à rotule oscillante étant relié de manière pivotante un bras oscillant et au moins la pale agitatrice étant montée dans la zone de l'extrémité libre de bras oscillant de sorte que des positions verticales différentes de la pale agitatrice soient réglées, associées en conséquence pour des positions pivotantes différentes du bras oscillant,
**caractérisée en ce que**
- une ouverture d'entretien (6) alignée verticalement, pouvant être fermée de manière étanche et détachable au moyen d'un couvercle d'entretien est disposée dans la zone supérieure de la paroi latérale de fermenteur (4),
- la au moins une articulation à rotule oscillante (12a, 12b) est montée de manière détachable dans la zone de bord latéral de l'ouverture d'entretien (6), de préférence sur un châssis-support (28) stable fixé dessus de telle manière qu'après le détachement d'au moins l'articulation à rotule oscillante (12a, 12b), le bras oscillant (14) puisse être déplacé du réservoir de fermenteur (2) à l'aide de l'entraînement rotatif (10) monté sur celui-ci et de la pale agitatrice (11) au travers de l'ouverture d'entretien (6),
- avant le détachement de la au moins une articulation à rotule oscillante (12a, 12b) au travers de l'ouverture d'entretien (6) de l'extérieur dans le réservoir de fermenteur (2), un dispositif d'appui (33, 35) peut être mis en place de préférence sur une extrémité avant de tracteur pour maintenir le bras oscillant (14) dans une position à peu près horizontale et pour le déplacer du réservoir de fermenteur (2) après le détachement de la au moins une articulation à rotule oscillante (12a, 12b).

2. Installation de biogaz selon la revendication 1, **caractérisée en ce que** le réservoir de fermenteur (2) est réalisé avec un toit de feuilles (5) comme élément de recouvrement de fermenteur.

3. Installation de biogaz selon la revendication 1 ou 2, **caractérisée en ce que** le bras oscillant (14) est réalisé comme un cadre oscillant rectangulaire ou triangulaire, une partie de cadre (30) étant logée de manière pivotante sur deux articulations à rotule oscillantes (12a, 12b) disposées coaxialement de manière espacée.

4. Installation de biogaz selon l'une quelconque des revendications 1 à 3, **caractérisée en ce que** le dispositif d'actionnement présente une unité à pistons/cylindres de réglage hydraulique et/ou pneumatique qui est articulée éventuellement de manière détachable d'une part sur le bras oscillant (14) et est articulée d'autre part sur la paroi latérale de fermenteur (4) sous le bras oscillant (14) éventuellement de manière à pouvoir se déplacer vers le haut sur un rail vertical (26).

5. Installation de biogaz selon l'une quelconque des revendications 1 à 4, **caractérisée en ce que** l'entraînement rotatif (10) est disposé comme un moteur d'entraînement électrique, pneumatique ou de préférence hydraulique sur l'extrémité du bras oscillant ou sur l'extrémité de la branche du bras oscillant (15) conjointement avec la ou les pales agitatrices (11).
